# FASCICULE DE BREVET EUROPEEN

(11) **EP 3 615 107 B1**
(45) Date de publication et mention de la délivrance du brevet: **14.08.2024**
(21) Numéro de dépôt: 18726518.6
(22) Date de dépôt: 23.04.2018
(51) Int. Cl.: A61M 5/14, A61M 5/142, A61M 5/145

(54) **DISPOSITIF D'INJECTION AUTOMATIQUE DE PRODUIT FLUIDE**
AUTOMATISCHE INJEKTIONSVORRICHTUNG FÜR FLUIDPRODUKTE
AUTOMATIC FLUID PRODUCT INJECTION DEVICE

(30) Priorité: 26.04.2017 FR 1753611
(43) Date de publication de la demande: 04.03.2020
(73) Titulaire: APTAR France SAS, 27110 Le Neubourg (FR)
(72) Inventeur: REEVES, Sam, Cambridge Cambridgeshire CB4 0WS (GB)
(74) Mandataire: CAPRI
(86) Numéro de dépôt international: PCT/FR2018/051010
(87) Numéro de publication internationale: WO 2018/197792

(56) Documents cités:
- WO-A2-02/40083
- WO-A2-03/090509
- WO-A2-2013/016376
- GB-A- 2 434 548

## Description

La présente invention concerne un dispositif d'injection automatique de produit fluide.

Les dispositifs d'injection automatique de produit fluide sont bien connus. Ils comportent notamment les autoinjecteurs, dans lesquels le contenu d'un réservoir, généralement une seringue, est automatiquement injecté au moyen d'un système d'actionnement comprenant généralement un ressort chargé et qui lors du déclenchement va déplacer un piston dans le réservoir pour injecter le produit fluide.

Ces dispositifs de l'art antérieur peuvent présenter des problèmes, notamment lorsque les volumes à distribuer sont importants, lorsque le produit fluide est relativement visqueux ou lorsque plusieurs produits fluides doivent être associés dans un même traitement.

Le document WO0240083 décrit un dispositif selon le préambule de la revendication 1.

La présente invention a pour but de fournir un dispositif d'injection automatique qui ne reproduit pas les inconvénients susmentionnés.

La présente invention a aussi pour but de fournir un dispositif d'injection automatique qui permet la distribution automatique de produit fluide, même à des volumes et/ou viscosités importants.

La présente invention a également pour but de fournir un dispositif d'injection automatique de produit fluide qui est simple et peu coûteux à fabriquer et à assembler.

La présente a donc pour objet un dispositif d'injection automatique de produit fluide comportant:
- un corps supérieur comportant au moins un système d'injection commandé par des moyens d'alimentation de puissance et déclenché par une interface utilisateur, tel qu'au moins un bouton d'actionnement, et un mécanisme d'insertion d'une aiguille d'injection,
- un corps de base, destiné à venir au contact d'une zone à injecter, comportant au moins un réservoir de produit fluide, contenant chacun un piston d'injection et un ensemble d'aiguille comportant ladite aiguille d'injection,
dans lequel ledit système d'injection comprend:
- au moins une bande métallique à ressort allongée, chaque bande étant avant actionnement enroulée sur une bobine respective, et
- un mécanisme d'entraînement pour dérouler ladite au moins une bande contre un piston respectif pour réaliser l'injection, et pour enrouler à nouveau ladite bande sur ladite bobine après injection.

Avantageusement, à l'état non enroulé, ladite bande est incurvée le long de son axe longitudinal.

Avantageusement, chaque bande est alimentée par un guide de sorte que l'extrémité libre de ladite bande est en position correcte pour s'engager avec ledit piston du réservoir respectif.

Avantageusement, ledit mécanisme d'entraînement comprend un moteur électrique et un entraînement à vis sans fin.

Avantageusement, ledit mécanisme d'insertion comporte un actionneur d'insertion et des moyens de déplacement d'aiguille, tel qu'un plongeur.

Avantageusement, ledit corps de base comporte une canalisation de distribution, comportant une aiguille d'amorçage respective pour chaque réservoir.

Avantageusement, le dispositif comporte des moyens pour amorcer ledit dispositif lors de l'assemblage dudit corps supérieur sur ledit corps de base.

Avantageusement, le dispositif comporte des moyens pour assurer que ledit mécanisme d'insertion ne peut être actionné pour insérer l'aiguille d'injection dans la zone à injecter qu'après amorçage.

Avantageusement, le dispositif comporte des moyens pour assurer que ledit système d'injection ne peut être actionné qu'après que l'aiguille d'injection a pénétré dans la zone à injecter.

Avantageusement, le dispositif comporte des moyens pour assurer que ledit mécanisme d'insertion ne peut être actionné pour rétracter l'aiguille d'injection hors de la zone à injecter qu'après la fin de l'injection.

Avantageusement, le dispositif comporte des moyens de contrôle mécanique et/ou logiciel pour déterminer la fin de l'injection, notamment des moyens de contrôle de la rotation d'une bobine.

Avantageusement, ledit corps de base comporte un autocollant pour se fixer sur la zone à injecter

Avantageusement, ledit corps de base comporte au moins deux réservoirs, notamment trois.

Avantageusement, chaque réservoir contient entre 1 et 10 ml de produit fluide, avantageusement environ 3 ml.

Avantageusement, lesdits moyens d'alimentation de puissance comportent une pile et/ou un accumulateur d'énergie électrique.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique en perspective éclatée d'un dispositif d'injection automatique selon un mode de réalisation avantageux,
- la figure 2 est une vue schématique de côté du dispositif de la figure 1,
- les figures 3 et 4 sont des vues schématiques en coupe du dispositif des figures 1 et 2, respectivement avant et après amorçage,
- les figures 5 à 7 sont des vues schématiques en coupe du dispositif des figures 1 et 2, respectivement avant injection, après injection et après rétractation de l'aiguille d'injection,
- les figures 8 et 9 sont des vues schématiques de détail en perspective d'un mécanisme d'injection selon un mode de réalisation avantageux, respectivement avant et après injection, et

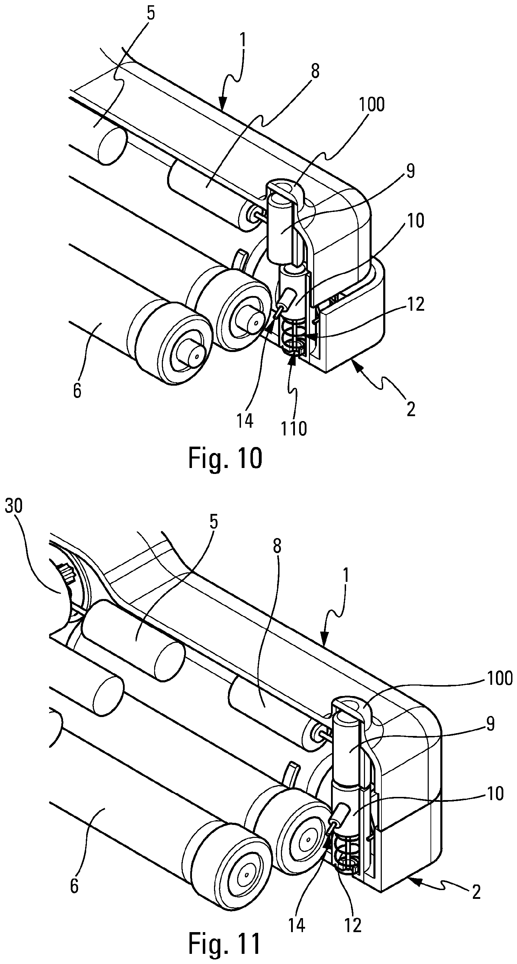

- les figures 10 à 13 sont des vues schématiques de côté de l'ensemble d'aiguille, respectivement avant amorçage, avant piquage, après injection et après rétractation de l'aiguille.

L'invention concerne un dispositif d'injection automatique particulièrement adapté à distribuer des volumes relativement importants de produit fluide, typiquement de l'ordre de quelques millilitres, typiquement de 1 à 10 ml, par exemple 3 ml. Le dispositif de l'invention est aussi adapté à distribuer des produits fluides relativement visqueux.

Le dispositif comprend deux parties distinctes.

Le corps de base 2 contient le ou les réservoir(s) 6 de médicament, chacun pourvu d'un piston d'injection 65 et d'une membrane de fermeture ou septum 61, l'ensemble d'aiguille 10, comprenant une aiguille d'injection 110, et une canalisation de distribution 11, comportant une aiguille d'amorçage 111 respective pour chaque réservoir 6, ladite canalisation de distribution 11 reliant, après amorçage, chaque réservoir 6 audit ensemble d'aiguille 10. Ledit corps de base 2 est de préférence jetable.

Le corps supérieur 1 contient le système d'injection 3, 4, 5, la source d'alimentation 7, le mécanisme d'insertion 8, 9 de l'aiguille d'injection 110, et l'interface utilisateur 100, en particulier un bouton d'actionnement. Ce corps supérieur 1 peut être jetable, mais de préférence il est réutilisable. Il est notamment envisageable que différents corps supérieurs 1, par exemple adaptées pour différents utilisateurs, peuvent coopérer avec un même corps de base 2.

Avantageusement, le dispositif est présenté à l'utilisateur avec le ou les réservoir(s) 6 de médicament(s) préassemblé(s) dans le corps de base 2.

Les étapes d'utilisation suivantes,qui ne font pas partie de l'invention, sont possibles:
1) L'utilisateur retire l'emballage et assemble les deux parties de l'appareil, à savoir le corps supérieur 1 et le corps de base 2;
2) L'utilisateur retire le support auto-adhésif du corps de base et fixe le dispositif sur le site d'injection;
3) L'utilisateur actionne le dispositif, notamment via le bouton d'actionnement 100, pour insérer l'aiguille 110, réaliser l'injection et rétracter l'aiguille 110;
4) L'utilisateur, après l'achèvement du processus d'injection, retire le dispositif du site d'injection;
5) L'utilisateur sépare les deux parties de l'appareil et de préférence ne jette que le corps de base.

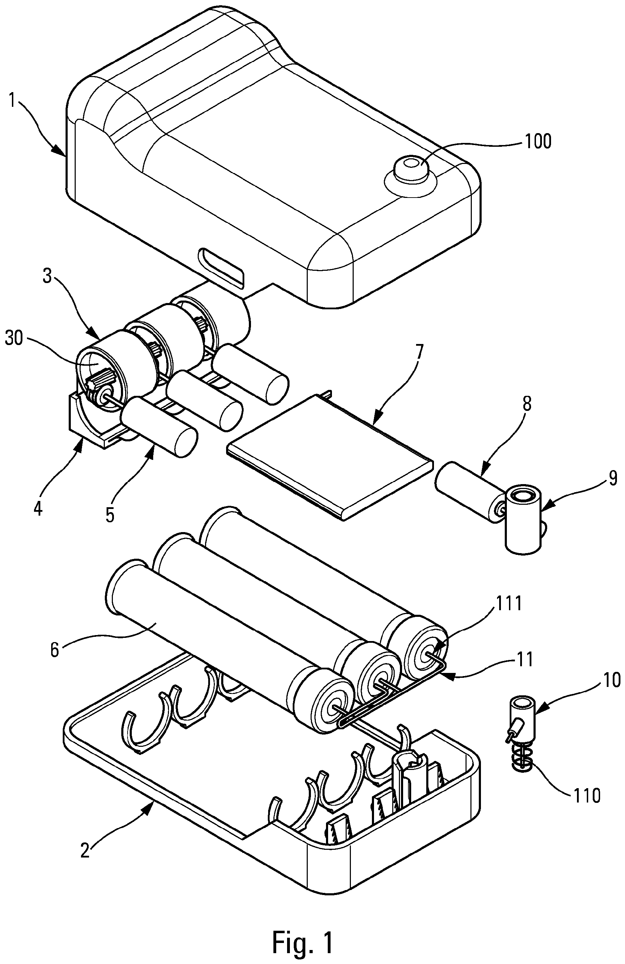

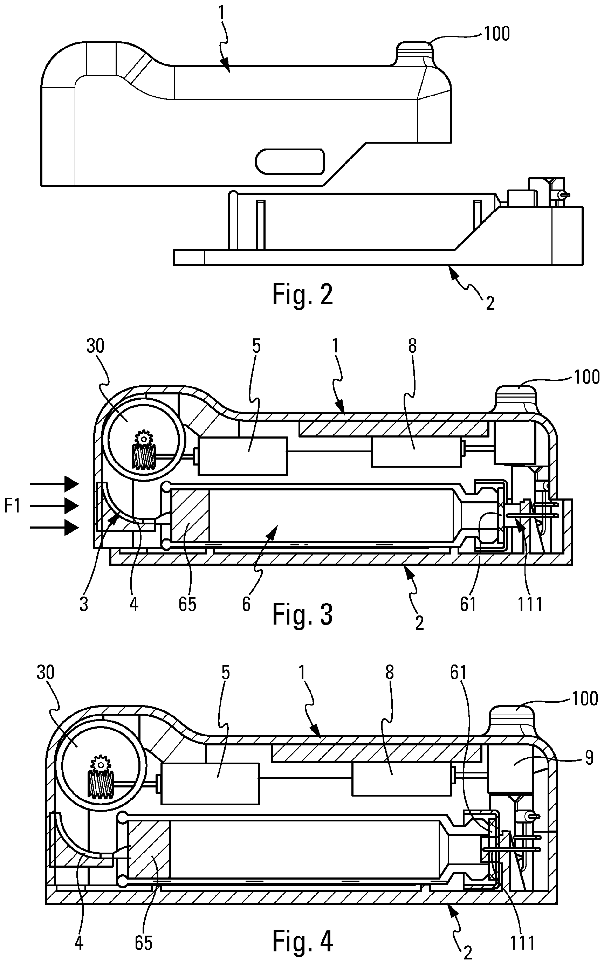

Sur les figures 1 et 2, il est représenté un dispositif d'injection automatique selon un mode de réalisation avantageux.

Dans l'exemple représenté, la source d'alimentation 7 est une pile ou un accumulateur d'énergie électrique.

Avant d'utiliser l'appareil, le patient doit assembler les corps supérieur 1 et de base 2 de l'appareil.

Ce faisant, les réservoirs 6 sont déplacées dans ledit corps de base 2 par rapport aux aiguilles d'amorçage 111 qui elles restent fixes dans le corps de base 2 lors de l'assemblage. De cette manière, chaque aiguille d'amorçage 111 perce le septum 61 d'un réservoir 6 respectif. Ainsi, lorsque les deux parties de l'appareil sont assemblées, celui-ci est amorcé et prêt à l'emploi.

Plus précisément, le ou les réservoir(s) 6 et l'ensemble d'aiguille 10 sont logés dans le corps de base 2 du dispositif. Dans l'état avant assemblage, les réservoirs 6 sont dans une position rétractée par rapport aux aiguilles d'amorçage 111 et les septums 61 sont intacts. Lorsque les corps supérieur 1 et de base 2 sont assemblés, un mécanisme d'entraînement de bande 3, faisant partie du système d'injection, s'engage avec l'arrière de chaque réservoir 6. Lors de l'assemblage, le corps supérieur 6 coulisse latéralement (vers la droite dans l'exemple des figures 3 et 4, comme représenté par les flèches F1) par rapport au corps de base 2 afin de se verrouiller en place. Ce faisant, les réservoirs 6 sont poussées contre les aiguilles d'amorçage 111 et les septums 61 sont percés.

Si plusieurs réservoirs 6 sont utilisés, comme représenté dans l'exemple des figures, les aiguilles d'amorçage 111 de tous les réservoirs 6 sont couplées à une seule aiguille d'injection 110.

Le mode de réalisation décrit présente notamment l'avantage qu'il permet une combinaison de composants jetables et réutilisables. Les composants qui entrent en contact avec le médicament sont logés dans le corps de base 2 et sont jetables. Les composants du système d'injection, du système d'insertion de l'aiguille d'injection et de l'interface utilisateur sont logés dans le corps supérieur 1, et peuvent donc être réutilisables.

L'aiguille d'injection 110 est insérée dans le site d'injection au moyen d'un actionneur d'insertion 8, notamment un actionneur mécanique, agissant sur un plongeur d'aiguille 9, et rétractée au moyen d'un ressort de rappel 12. En variante, l'actionneur d'insertion 8 pourrait être un actionneur électromécanique, comportant par exemple un moteur associé à une vis sans fin. D'autres variantes sont envisageables.

De préférence, des moyens sont prévus pour assurer que l'aiguille d'injection 110 ne peut être déployée qu'après amorçage du dispositif. Ceci peut être identifié par un contrôle mécanique et/ou logiciel, par exemple par un contrôle de la position relative des corps supérieur 1 et de base 2. Par exemple, lorsque les deux corps 1, 2 sont assemblés entre eux pour amorcer l'appareil, le plongeur 9 de l'aiguille d'injection 110 vient s'aligner avec l'ensemble d'aiguille 10; par conséquent, le déploiement de l'aiguille 110 ne peut pas avoir lieu avant l'amorçage.

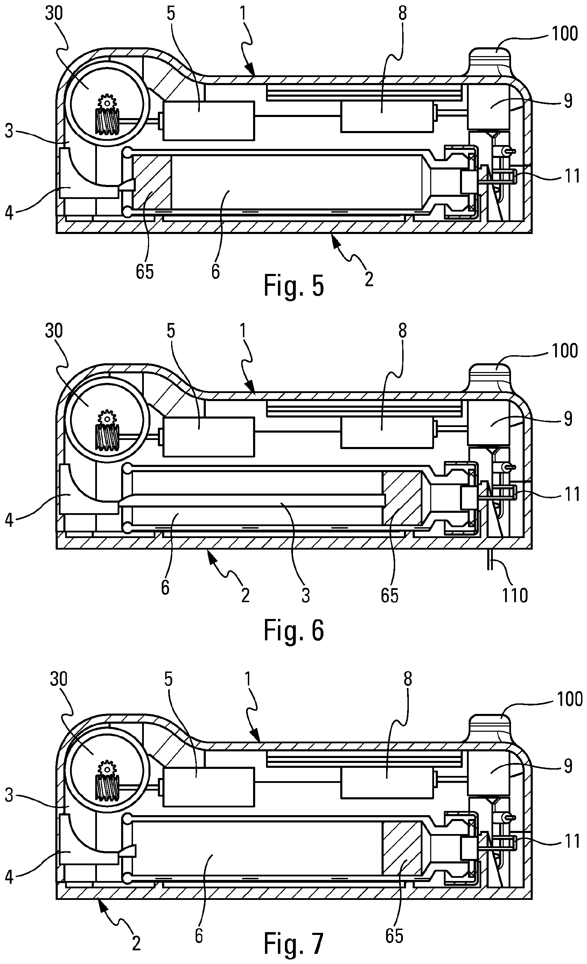

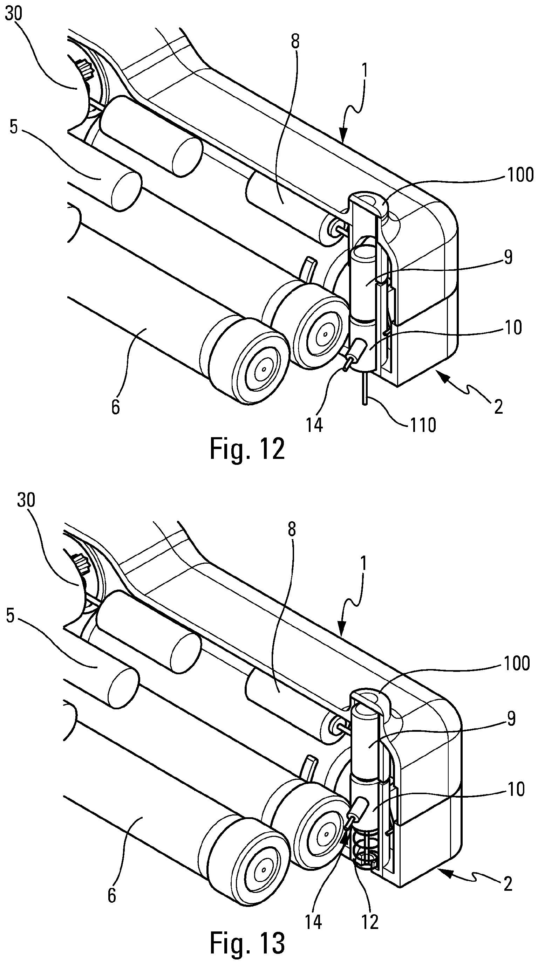

Lorsque le dispositif est actionné, par exemple via le bouton d'actionnement 100, l'actionneur d'insertion 8 enfonce le plongeur 9 qui, à son tour, entraîne l'aiguille d'injection 110 dans le site d'injection, comme visible sur les figures 6 et 12.

Lorsque l'injection est terminée, l'actionneur d'insertion 8 rétracte le plongeur 9, et le ressort de rappel 12 retire l'aiguille du site d'injection, comme visible sur les figures 7 et 13. La fin de l'injection peut être identifiée par des moyens de contrôle mécanique et/ou logiciel, par exemple en surveillant la rotation d'une bobine 30 de bande 3.

Le mode de réalisation décrit présente notamment l'avantage que les composants mécaniques du mécanisme d'insertion sont séparés des composants de l'ensemble d'aiguille 10 qui entrent en contact avec le patient/médicament. Cela leur permet au mécanisme d'insertion d'être réutilisable.

Le système d'injection comprend au moins une bande métallique à ressort allongée, typiquement en acier à ressort. Dans sa forme libre, c'est-à-dire lorsqu'elle n'est pas enroulée sur une bobine, la bande 3 est incurvée le long de son axe longitudinal pour créer une certaine rigidité. Lorsqu'elle est aplatie, la bande 3 peut être enroulée sur une bobine 30 de façon à occuper un petit volume pendant le stockage.

Le dispositif peut comporter une ou plusieurs bandes 3 pour distribuer le contenu d'un ou plusieurs réservoirs.

Chaque bande 3 est alimentée par un guide 4 de sorte que l'extrémité libre de la bande 3 est en position correcte pour s'engager avec le piston 65 du réservoir 6 respectif.

Pendant le processus d'injection, les bobines 30 de bande sont tournées au moyen d'un mécanisme d'entraînement 5. Dans le mode de réalisation représenté ici, celui-ci comprend un moteur électrique et un entraînement à vis sans fin. D'autres variantes appropriées sont envisageables.

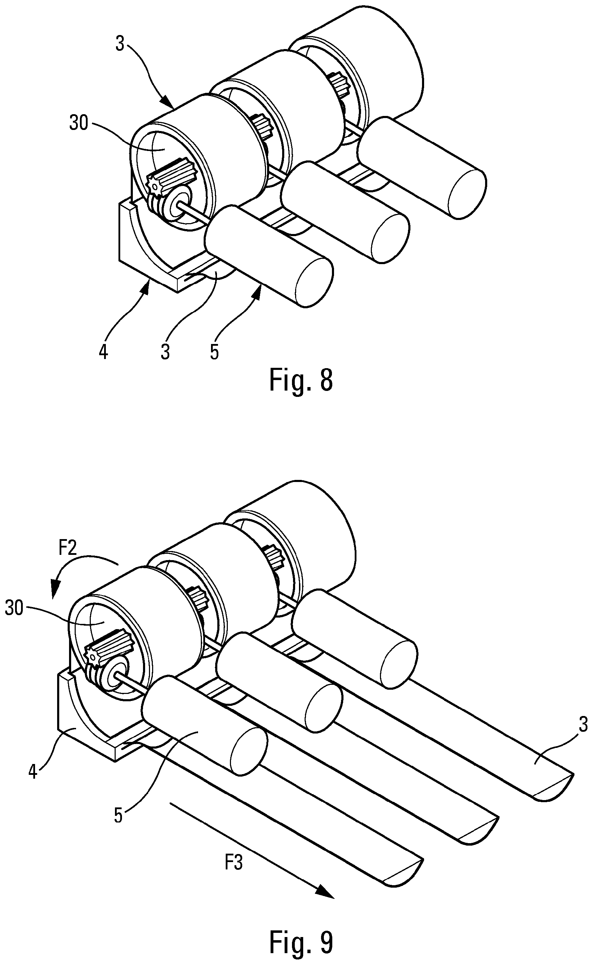

Lorsque la bobine 30 de bande tourne, comme illustré par la flèche F2 sur la figure 9, la bande 3 est forcée dans le guide 4 et contre le piston 65 du réservoir 6. Lorsque la bande 3 sort du guide, elle revient à sa forme courbe, comme visible sur la figure 9. Ceci crée une longueur de bande sensiblement rigide adaptée à pousser le piston 65 du réservoir 6, comme représenté par la flèche F3.

Lorsque l'injection du ou des médicament(s) est terminée, le mécanisme d'entraînement 5 est inversé et la bande 3 est rétractée, donc enroulée à nouveau sur sa bobine 30.

Le mode de réalisation décrit présente notamment l'avantage que le système d'injection est réversible donc réutilisable. De plus, il présente une forme compacte, de par l'enroulement à plat de la ou des bande(s) 3 sur la ou les bobine(s) 30.

Avantageusement, le mécanisme d'entrainement 5 du système d'injection ne peut être actionné qu'après que l'aiguille d'injection 110 a pénétré dans le site d'injection. Ceci peut être réalisé au moyen d'un contrôle mécanique et/ou logiciel, par exemple en analysant la position du plongeur 9 du mécanisme d'insertion de l'aiguille.

Le dispositif représenté sur les figures comporte trois systèmes d'injection agissant sur trois réservoirs 6. Les trois systèmes d'injection peuvent être actionnés simultanément, au moyen d'un seul bouton d'actionnement 100, pour distribuer simultanément les contenus des trois réservoirs, qui sont alors mélangés en amont de l'aiguille d'injection 110. En variante, les trois systèmes d'injection peuvent être actionnés successivement, pour distribuer successivement les contenus des trois réservoirs. Ces actionnements successifs peuvent être déclenchés au moyen de boutons d'actionnement distincts, mais on pourrait aussi prévoir un seul bouton d'actionnement qui déclenche automatiquement la séquence de distribution. Un mélange de ces deux variantes est aussi possible, par exemple une distribution en deux temps, avec d'une part la distribution du contenu d'un réservoir et d'autre part la distribution simultanée du mélange des deux autres réservoirs.

Le mode de réalisation décrit fournit notamment les avantages suivants:
- la vitesse d'injection du produit fluide peut être ajustée pour optimiser les traitements individuels et peut également varier dans le temps;
- les systèmes d'injection et les réservoirs multiples permettent l'utilisation d'une combinaison de médicaments qui peuvent être distribués à des vitesses différentes et à des moments différents.

L'utilisation d'un dispositif à un ou plusieurs réservoirs permet de fournir notamment les avantages suivants:
- dispositif unique pour plusieurs types de produits fluides, qui peut nécessiter la distribution de volumes différents;
- possibilité de distribuer des cocktails ou mélange de plusieurs produits fluides;
- possibilités d'associer des agents réducteurs de douleur (anesthésiques, neutralisateur d'acidité, etc.) au médicament à injecter;
- possibilité d'avoir des fréquences de traitement de médicaments différents; par exemple une première séquence S1 avec la prise de plusieurs médicaments différents, suivie d'une seconde séquence S2 avec la prise d'un seul médicament, etc.;
- possibilité de standardiser le dispositif d'injection pour plusieurs types de traitement;
- diminution du coût de développement des dispositifs;
- possibilité d'ajustements dans la formulation du produit fluide.
- diverses formulations de produits fluides peuvent être logées dans un seul dispositif;
- réduction du nombre d'injections.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Dispositif d'injection automatique de produit fluide comportant:
- un corps supérieur (1) comportant au moins un système d'injection (3, 4, 5) commandé par des moyens d'alimentation de puissance (7) et déclenché par une interface utilisateur (100), tel qu'au moins un bouton d'actionnement, et un mécanisme d'insertion (8, 9) d'une aiguille d'injection (110),
- un corps de base (2), destiné à venir au contact d'une zone à injecter, comportant au moins un réservoir (6) de produit fluide, contenant chacun un piston d'injection (65) et un ensemble d'aiguille (10) comportant ladite aiguille d'injection (110),
**caractérisé en ce que** ledit système d'injection comprend:
- au moins une bande métallique à ressort allongée (3), chaque bande étant avant actionnement enroulée sur une bobine (30) respective, et
- un mécanisme d'entraînement (5) pour dérouler ladite au moins une bande (3) contre un piston (65) respectif pour réaliser l'injection, et pour enrouler à nouveau ladite bande (3) sur ladite bobine (30) après injection.

2. Dispositif selon la revendication 1, dans lequel, à l'état non enroulé, ladite bande (3) est incurvée le long de son axe longitudinal.

3. Dispositif selon la revendication 1 ou 2, dans lequel chaque bande (3) est alimentée par un guide (4) de sorte que l'extrémité libre de ladite bande (3) est en position correcte pour s'engager avec ledit piston (65) du réservoir (6) respectif.

4. Dispositif selon l'une quelconque des revendications précédentes, dans lequel ledit mécanisme d'entraînement (5) comprend un moteur électrique et un entraînement à vis sans fin.

5. Dispositif selon l'une quelconque des revendications précédentes, dans lequel ledit mécanisme d'insertion comporte un actionneur d'insertion (8) et des moyens de déplacement d'aiguille (9), tel qu'un plongeur.

6. Dispositif selon l'une quelconque des revendications précédentes, dans lequel ledit corps de base (2) comporte une canalisation de distribution (11), comportant une aiguille d'amorçage (111) respective pour chaque réservoir (6).

7. Dispositif selon la revendication 6, comportant des moyens pour amorcer ledit dispositif lors de l'assemblage dudit corps supérieur (1) sur ledit corps de base (2).

8. Dispositif selon l'une quelconque des revendications précédentes, comportant des moyens pour assurer que ledit mécanisme d'insertion (8, 9) ne peut être actionné pour insérer l'aiguille d'injection (110) dans la zone à injecter qu'après amorçage.

9. Dispositif selon l'une quelconque des revendications précédentes, comportant des moyens pour assurer que ledit système d'injection (3, 4, 5) ne peut être actionné qu'après que l'aiguille d'injection (110) a pénétré dans la zone à injecter.

10. Dispositif selon l'une quelconque des revendications précédentes, comportant des moyens pour assurer que ledit mécanisme d'insertion (8, 9) ne peut être actionné pour rétracter l'aiguille d'injection (110) hors de la zone à injecter qu'après la fin de l'injection.

11. Dispositif selon la revendication 10, comportant des moyens de contrôle mécanique et/ou logiciel pour déterminer la fin de l'injection, notamment des moyens de contrôle de la rotation d'une bobine (30).

12. Dispositif selon l'une quelconque des revendications précédentes, dans lequel ledit corps de base (2) comporte un autocollant pour se fixer sur la zone à injecter

13. Dispositif selon l'une quelconque des revendications précédentes, dans lequel ledit corps de base (2) comporte au moins deux réservoirs (6), notamment trois.

14. Dispositif selon l'une quelconque des revendications précédentes, dans lequel chaque réservoir contient entre 1 et 10 ml de produit fluide, avantageusement environ 3 ml.

15. Dispositif selon l'une quelconque des revendications précédentes, dans lequel lesdits moyens d'alimentation de puissance (7) comportent une pile et/ou un accumulateur d'énergie électrique.

## Patentansprüche

1. Vorrichtung zur automatischen Injektion von Flüssigprodukten, Folgendes umfassend:
- einen oberen Körper (1) mit mindestens einem Injektionssystem (3, 4, 5), das durch Stromversorgungsmittel (7) gesteuert und durch eine Benutzerschnittstelle (100) ausgelöst wird, beispielsweise mindestens eine Betätigungstaste, und einen Mechanismus (8, 9) zum Einführen einer Injektionsnadel (110),
- einen Grundkörper (2), der dafür bestimmt ist, mit einer zu injizierenden Stelle in Kontakt zu kommen, mit mindestens einem Vorratsbehälter (6) für ein Flüssigprodukt, der jeweils einen Injektionskolben (65) und eine die Injektionsnadel (110) umfassende Nadelanordnung (10) enthält,
**dadurch gekennzeichnet, dass** das Injektionssystem Folgendes umfasst:
- mindestens ein längliches Federmetallband (3), wobei jedes Band vor der Betätigung auf eine entsprechende Spule (30) aufgewickelt ist, und
- einen Antriebsmechanismus (5) zum Abwickeln des mindestens einen Bandes (3) gegen einen entsprechenden Kolben (65), um die Injektion durchzuführen, und zum erneuten Aufwickeln des Bandes (3) auf die Spule (30) nach erfolgter Injektion.

2. Vorrichtung nach Anspruch 1, bei der das Band (3) im nicht aufgewickelten Zustand entlang seiner Längsachse gebogen ist.

3. Vorrichtung nach Anspruch 1 oder 2, bei der jedes Band (3) durch eine Führung (4) zugeführt wird, sodass sich das freie Ende des Bandes (3) in der richtigen Position befindet, um mit dem Kolben (65) des jeweiligen Vorratsbehälters (6) in Eingriff zu kommen.

4. Vorrichtung nach einem der vorhergehenden Ansprüche, bei der der Antriebsmechanismus (5) einen Elektromotor und einem Schneckenantrieb umfasst.

5. Vorrichtung nach einem der vorhergehenden Ansprüche, bei der der Einführmechanismus ein Einführbetätigungselement (8) und Nadelbewegungsmittel (9), beispielsweise einen Stößel, umfasst.

6. Vorrichtung nach einem der vorhergehenden Ansprüche, bei der der Grundkörper (2) eine Verteilerleitung (11) aufweist, die eine jeweilige Auslösenadel (111) für jeden Vorratsbehälter (6) umfasst.

7. Vorrichtung nach Anspruch 6 mit Mitteln zum Auslösen der Vorrichtung beim Zusammenbau des Oberkörpers (1) mit dem Grundkörper (2).

8. Vorrichtung nach einem der vorhergehenden Ansprüche, umfassend Mittel zum Sicherstellen, dass eine Betätigung des Einführmechanismus (8, 9) zur Einführung der Injektionsnadel (110) in die zu injizierende Stelle erst nach erfolgter Auslösung möglich ist.

9. Vorrichtung nach einem der vorhergehenden Ansprüche, umfassend Mittel zum Sicherstellen, dass eine Betätigung des Injektionssystems (3, 4, 5) erst nach erfolgtem Eindringen der Injektionsnadel (110) in die zu injizierende Stelle möglich ist.

10. Vorrichtung nach einem der vorhergehenden Ansprüche, umfassend Mittel zum Sicherstellen, dass eine Betätigung des Einführmechanismus (8, 9) zum Herausziehen der Injektionsnadel (110) aus der zu injizierenden Stelle erst nach Beendigung der Injektion möglich ist.

11. Vorrichtung nach Anspruch 10, umfassend mechanische und/oder softwarebasierte Steuermittel zur Bestimmung des Endes der Injektion, insbesondere Steuermittel zur Steuerung der Drehung einer Spule (30).

12. Vorrichtung nach einem der vorhergehenden Ansprüche, bei der der Grundkörper (2) einen Aufkleber zu dessen Anbringung an der zu injizierenden Stelle umfasst.

13. Vorrichtung nach einem der vorhergehenden Ansprüche, bei der der Grundkörper (2) zwei Vorratsbehälter (6), insbesondere drei, aufweist.

14. Vorrichtung nach einem der vorhergehenden Ansprüche, bei der jeder Vorratsbehälter 1 ml bis 10 ml an Flüssigprodukt enthält, vorteilhafterweise etwa 3 ml.

15. Vorrichtung nach einem der vorhergehenden Ansprüche, bei der die Stromversorgungsmittel (7) eine Batterie und/oder einen elektrischen Energiespeicher umfassen.

## Claims

1. An automatic fluid injection device comprising:
- an upper body (1) comprising: at least one injection system (3, 4, 5) that is controlled by power supply means (7) and that is triggered by a user interface (100), such as at least one actuator button; and an insertion mechanism (8, 9) for inserting an injection needle (110),
- a base body (2) for coming into contact with an injection zone and comprising: at least one fluid reservoir (6), each containing an injection piston (65); and a needle assembly (10) including said injection needle (110),
said device being **characterized in that** said injection system comprises:
- at least one elongate spring metal strip (3), each strip being wound on a respective reel (30) before actuation,
- a drive mechanism (5) for unwinding said at least one strip (3) against a respective piston (65) so as to perform the injection, and for rewinding said strip (3) on said reel (30) after injection.

2. A device according to claim 1, wherein, in its unwound state, said strip (3) is curved along its longitudinal axis.

3. A device according to claim 1 or claim 2, wherein each strip (3) is delivered via a guide (4) so that the free end of said strip (3) is in its correct position for engaging with said piston (65) of the respective reservoir (6).

4. A device according to any preceding claim, wherein said drive mechanism (5) comprises an electric motor and a worm gear drive.

5. A device according to any preceding claim, wherein said insertion mechanism comprises an insertion actuator (8) and needle movement means (9), such as a plunger.

6. A device according to any preceding claim, wherein said base body (2) includes a dispenser pipe (11), including a respective priming needle (111) for each reservoir (6).

7. A device according to claim 6, including means for priming said device while assembling said upper body (1) on said base body (2).

8. A device according to any preceding claim, including means for guaranteeing that said insertion mechanism (8, 9) can be actuated in order to insert the injection needle (110) into the injection zone after priming.

9. A device according to any preceding claim, including means for guaranteeing that said injection system (3, 4, 5) can be actuated only after the injection needle (110) has penetrated into the injection zone.

10. A device according to any preceding claim, including means for guaranteeing that said insertion mechanism (8, 9) can be actuated in order to retract the injection needle (110) out from the injection zone only after the end of injection.

11. A device according to claim 10, including mechanical and/or software verification means for determining the end of injection, in particular verification means for verifying the turning of a reel (30).

12. A device according to any preceding claim, wherein said base body (2) includes a sticker for fastening onto the injection zone.

13. A device according to any preceding claim, wherein said base body (2) includes at least two reservoirs (6), in particular three reservoirs.

14. A device according to any preceding claim, wherein each reservoir has a fluid content in the range 1 mL to 10 mL, advantageously about 3 mL.

15. A device according to any preceding claim, wherein said power supply means (7) comprise an optionally rechargeable battery.
